# EUROPÄISCHE PATENTANMELDUNG

(11) **EP 0 638 291 A2**
(43) Veröffentlichungstag der Anmeldung: **15.02.1995**
(21) Anmeldenummer: 94110756.7
(22) Anmeldetag: 11.07.1994
(51) Int. Cl.: A61B 17/39

(54) **Bipolares Hochfrequenz-Chirurgieinstrument**

(30) Priorität: 14.07.1993 DE 4323585
(71) Anmelder: DELMA ELEKTRO-UND MEDIZINISCHE APPARATEBAU GESELLSCHAFT mbH, D-78532 Tuttlingen (DE)
(72) Erfinder: Fritzsch, Gernod, D-78532 Tuttlingen (DE)
(74) Vertreter: Dipl.-Phys.Dr. Manitz Dipl.-Ing. Finsterwald Dipl.-Ing. Grämkow Dipl.-Chem.Dr. Heyn Dipl.-Phys. Rotermund Morgan B.Sc.(Phys.)

(57) **Zusammenfassung**

Die Erfindung betrifft ein bipolares Hochfrequenz-Chirurgieinstrument mit einem starren Rohrschaft (11) und einer vorzugsweise eine im wesentlichen den gleichen Querschnitt wie der anschließende Teil des Rohrschaftes (11) aufweisenden Arbeitsspitze (12), an der zentral wenigstens eine von einer Hochfrequenzspannung beaufschlagbare Arbeitselektrode, wie eine Schneidelektrode (13, 13") oder Koagulationselektrode (13') und eine vorzugsweise eine deutlich größere Oberfläche aufweisende Neutralelektrode (14) vorgesehen sind. Die Arbeitsspitze (12) ist nahe dem distalen Ende des Instrumentes gelenkig oder biegsam mit dem starren Rohrschaft (11) derart verbunden, daß sie aus einer mit dem Rohrschaft (11) axial ausgerichteten Position in eine relativ zum Rohrschaft (11) abgewinkelte Position verschwenkbar ist, und daß Mittel (17, 18, 19) vorgesehen sind, um die Arbeitsspitze (12) zu verschwenken und wieder zurückzustellen.

## Beschreibung

Die Erfindung betrifft ein bipolares Hochfrequenz-Chirurgieinstrument nach dem Oberbegriff des Patentanspruchs 1.

Derartige bipolare Hochfrequenz-Chirurgieinstrumente weisen im allgemeinen einen starren Schaft auf, an dessen distalem Ende eine Neutralelektrode und relativ dazu isoliert eine Schneid-und/oder Koagulationselektrode vorgesehen ist. Ein Problem bei der Handhabung derartiger Instrumente besteht darin, daß der Schneid- oder Koagulationsvorgang vom Operateur insbesondere dann nur schwer zu beobachten ist, wenn das Instrument zusammen mit einem Beobachtungs-Endoskop durch ein Trokar zur Operationsstelle geführt wird. Die Blickrichtung des Operateurs ist dabei annähernd die gleiche wie die Richtung der Achse des Rohrschaftes des Chirurgieinstrumentes, so daß die Stelle, wo die Schneid- oder Koagulationselektrode mit dem zu behandelnden Gewebe in Kontakt kommt, nur schwer oder garnicht beobachtbar ist.

Aus diesem Grunde hat man schon den Schneiddraht am distalen Ende der Arbeitsspitze seitlich soweit abgebogen, daß er über den Außenumfang des Rohrschaftes etwas vorsteht und somit bei in etwa axialer Blickrichtung vom Operateur beobachtbar ist. Diese Ausbildung hat jedoch den Nachteil, daß die Einführung des Instrumentes durch das Trokar und das Hinführen zur Operationsstelle durch die seitlich vorstehende Schneidelektrode erschwert ist und die empfindliche Schneidelektrode dabei beschädigt werden kann.

Eine entsprechende Ausbildung eines Koagulationselektrodenpaares ist bisher nicht bekanntgeworden.

Das Ziel der Erfindung besteht somit darin, ein bipolares Hochfrequenz-Chirurgieinstrument der eingangs genannten Gattung zu schaffen, welches sich insbesondere für die laparoskopische Chirurgie eignet, d.h. problemlos durch ein Trokar zum Operationsfeld fuhrbar ist, gleichwohl aber eine einwandfreie Beobachtung des Koagulations- oder Schneidvorganges durch den Operateur auch bei annähernd axialer Blickrichtung gestattet.

Zur Lösung dieser Aufgabe sind die Merkmale des kennzeichnenden Teils des Patentanspruchs 1 vorgesehen.

Aufgrund der erfindungsgemäßen Ausbildung kann das Instrument wahlweise mit mit dem Rohrschaft axial ausgerichteter oder abgewinkelter Arbeitsspitze verwendet werden. Beim Einführen bzw. Herausziehen durch ein Trokar wird die Arbeitsspitze erfindungsgemäß mit dem Rohrschaft ausgerichtet, so daß am distalen Ende keine seitlich vorstehenden Teile vorhanden sind, die die Bewegung des Instruments innerhalb des Trokars oder im Körper der zu operierenden Person behindern. Im Bereich des Operationsfeldes kann dann die Arbeitsspitze ausgeschwenkt werden, wodurch sie in das Blickfeld des beispielsweise durch ein Endoskop die Operation beobachtenden Operateurs gelangt. Es ist zwar bekannt, das distale Ende von Endoskopen (DE 39 05 455 C2; EP 0 422 842 A2) oder Kathetern (EP 0 489 937 A1) gesteuert abbiegbar auszubilden, um damit auch Bereiche innerhalb des Körpers zu erreichen, an die man mit einem geradlinigen Instrument nicht gelangen könnte; Sinn und Zweck der erfindungsgemäßen Ausbildung bestehen jedoch nicht darin, sonst nicht zugängliche Bereiche innerhalb des menschlichen Körpers mit dem Chirurgieinstrument zu erreichen, sondern vielmehr den Operationsvorgang unmittelbar durch ein Endoskop beobachten zu können. Hierfür ist es von Bedeutung, daß die Drehachse der Arbeitsspitze nicht zu weit vom distalen Ende des Instrumentes entfernt ist. Mit anderen Worten soll bei in die Schneid- oder Koagulationsstellung ausgeschwenkter Arbeitsspitze die Schneid- oder Koagulationselektrode nur gerade so weit radial über den Außenumfang des starren Rohrschaftes hinaus vorstehen, daß der Operationsvorgang mittels der betreffenden Elektrode vom Operateur durch ein mit in das Trokar eingeführtes Endoskop oder durch das als Endoskop selbst ausgebildete Trokar unmittelbar beobachtet werden kann.

Bevorzugte Abstände der Drehachse vom distalen Ende des Instruments ergeben sich aus Anspruch 2.

Drei besonders bevorzugte Schwenkmöglichkeiten der Arbeitsspitze sind durch die Ansprüche 3 bis 5 gekennzeichnet.

Vorteilhafte Weiterbildungen der Ausführungsform nach Anspruch 5 entnimmt man den Ansprüchen 6 bis 8.

Für die Verstellung der Arbeitsspitze zwischen ihrer axial mit dem Rohrschaft ausgerichteten Ruheposition in die abgewinkelte Ausstellposition entnimmt man Anspruch 9 verschiedene Antriebe.

Die Mittel zum Verschwenken und Zurückstellen der Arbeitsspitze 12 sind vorzugsweise in der Weise mit Anschlägen versehen, daß die Arbeitsspitze nur zwei Positionen einnehmen kann, nämlich die mit dem geradlinigen Rohrschaft axial ausgerichtete Ruheposition oder die abgewinkelte Arbeitslage.

Eine weitere vorteilhafte Verstellmöglichkeit für die Arbeitsspitze ist durch Anspruch 10 gekennzeichnet.

Eine weitere vorteilhafte Ausführungsform entnimmt man Anspruch 11.

Das Federelement kann zweckmäßigerweise gemäß Anspruch 12 ausgebildet sein.

Zum Abtragen von Biogewebe ist die Ausführungsform nach den Ansprüchen 13 oder 14 von Vorteil. Für ein Schneidinstrument ist die Ausbildung nach Anspruch 15 zweckmäßig.

Besonders bevorzugt wird die Erfindung bei einem kombinierten Instrument nach Anspruch 16 angewendet, welches zweckmäßig gemäß Anspruch 17 ausgebildet ist.

Für die Schneidnadel gibt Anspruch 18 geeignete Materialien an.

Besonders vorteilhafte Schwenkwinkel der Arbeitsspitze in der Arbeitsposition entnimmt man Anspruch 19.

Die Erfindung wird im folgenden beispielsweise anhand der Zeichnung beschrieben; in dieser zeigt:
Fig. 1 eine schematische Teilseitenansicht einer ersten Ausführungsform eines erfindungsgemäßen Hochfrequenz-Chirurgie-Schneidinstrumentes,
Fig. 2 eine entsprechende Ansicht einer weiteren Ausführungsform,
Fig. 3 eine vergrößerte Teil-Seitenansicht einer ähnlichen Ausführungsform wie Fig. 2,
Fig. 4 eine entsprechende Ansicht wie die Fig. 1 und 2 einer weiteren Ausführungsform,
Fig. 5 eine schematische Seitenansicht einer weiteren Ausführungsform,
Fig. 6 eine schematische Seitenansicht einer mit einem axial verschiebbaren Steuerglied versehenen Ausführungsform,
Fig. 7 eine schematische Seitenansicht einer ähnlichen Ausführungsform wie Fig. 6, die
Fig. 8, 8a eine mit einem Drehverstellrohr arbeitende Ausführungsform in der Ruhe- bzw. Arbeitsposition, die
Fig. 9, 9a eine ähnliche Ausführungsform wie die Fig. 8, 8a ebenfalls in der Ruhe- bzw. Arbeitsposition,
Fig. 10 eine mit einer Schneidschlinge versehene Arbeitsspitze in einer schematischen, teilweise geschnittenen Seitenansicht, und die
Fig. 11, 11 geschnittene Seitenansichten einer weiteren Arbeitsspitze für ein erfindungsgemäßes Hochfrequenz-Chirurgieinstrument, welche sowohl das Koagulieren als auch das Schneiden ermöglicht, wobei in Fig. 11 die Schneidposition und in Fig.11 a die Koagulationsposition veranschaulicht ist.

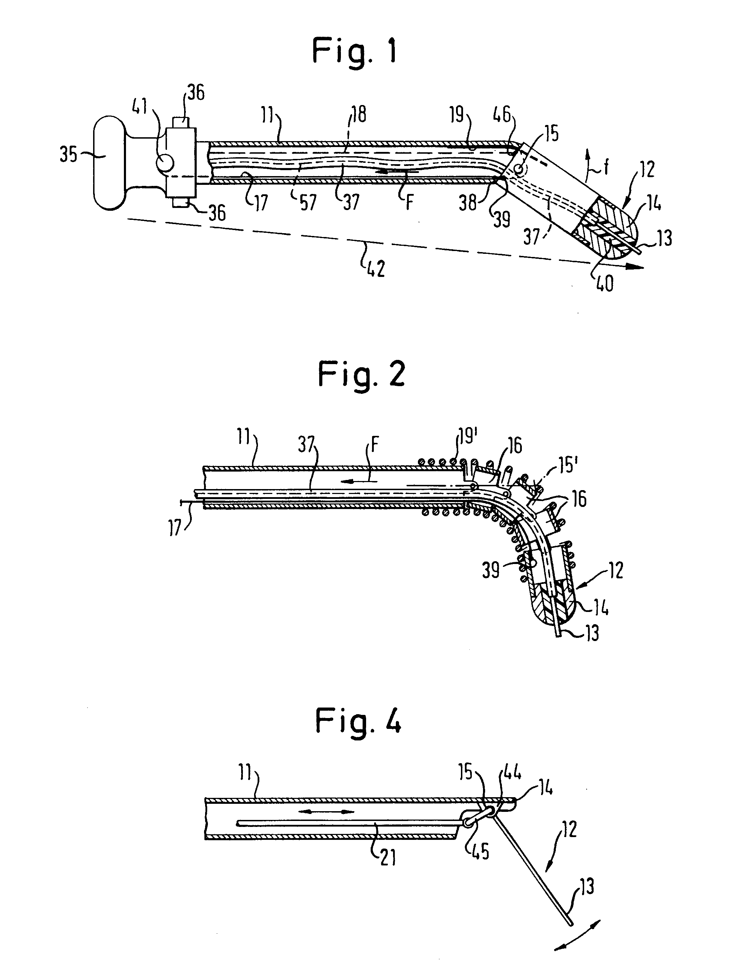

Nach Fig. 1 weist das erfindungsgemäße Hochfrequenz-Chirurgie-Schneidinstrumenteinen, vorzugsweise aus Metall bestehenden und geerdeten starren Rohrschaft 11 auf, der an seinem hinteren Ende einen nur schematisch angedeuteten Handgriff 35 mit Betätigungselementen 36, 41 für die verschiedenen Funktionen aufweist und in seinem Inneren ein isoliertes Hochfrequenzzuführungskabel 37 aufnimmt, welches die Hochfrequenzspannung von einem nicht dargestellten Hochfrequenzgenerator zur am distalen Ende des Instrumentes vorgesehenen Schneidelektrode 13 zuführt.

Am distalen Ende des vorzugsweise kreiszylindrischen Rohrschaftes 11 ist über eine quer zur Rohrschaftachse verlaufende Drehachse 15 eine Arbeitsspitze 12 angelenkt, welche vorzugsweise nicht länger als 2 bis 3 cm ist. Der Schwenkbereich der Arbeitsspitze 12 ist durch ein Anschlagflächenpaar 38 auf einen Winkel von etwa 30 zur Mittellängsachse des geradlinigen Rohrschaftes 11 beschränkt.

Vom distalen Endbereich des Rohrschaftes 11 in die vorzugsweise ebenfalls als Rohr ausgebildete Arbeitsspitze 12 erstreckt sich oberhalb der Drehachse 15 eine längliche Feder 19, die die Arbeitsspitze 12 relativ zum Rohrschaft in Richtung des Pfeiles f so vorspannt, daß sie normalerweise in einer Einführ- und Entnahmeposition mit dem Rohrschaft 11 ausgerichtet ist.

Zwischen einem Punkt 39 unterhalb der Drehachse 15 und einem Drehknopf 41 im proximalen Ende des Rohrschaftes 11 erstreckt sich ein Zugseil oder -draht 17, der in Richtung des Pfeiles F mit einer Zugkraft beaufschlagbar ist und so die Arbeitsspitze 12 gegen die rückstellende Kraft der Feder 19 in die aus Fig.1 ersichtliche Arbeitsposition schwenken kann.

Das isolierte Hochfrequenzkabel 37 erstreckt sich an der Drehachse 15 vorbei in die rohrförmige Arbeitsspitze 12 hinein, welche an ihrem distalen Ende eine konvex gekrümmte Neutralelektrode 14 aufweist, innerhalb der sich ein Isolierkern 40 befindet, in dem koaxial eine vorn vorstehende Schneidelektrode 13 gelagert ist, welche mit der gestrichelt angedeuteten Seele 37 des Hochfrequenzkabels 37 elektrisch leitend verbunden ist.

Die Arbeitsweise des in Fig.1 gezeigten Hochfrequenz-Chirurgieinstrumentes ist wie folgt:
Zunächst wird durch geeignete Betätigung des Drehknopfes 41 das in Fig. 1 gespannt dargestellte Zugseil 17 gelockert, so daß die Feder 19 die Arbeitsspitze 12 in Richtung des Pfeiles f in die mit dem Rohrschaft 11 ausgerichtete Einführ- und Entnahmeposition schwenken kann. In dieser Position wird das Instrument beispielsweise durch ein Trokar in eine Körperhöhle eingeführt, wo die Operation stattfinden soll. Sobald sich die Arbeitsspitze 12 im Bereich des Operationsfeldes befindet, betätigt der Operateur den Drehknopf 41, wodurch das Zugseil 17 in Richtung des Pfeiles F bewegt wird und dadurch die Arbeitsspitze 12 gegen die Kraft der Feder 19 in die aus Fig. 1 ersichtliche Arbeitsposition verschwenkt. Nunmehr befindet sich die von der Arbeitsspitze 12 vorstehende Schneidelektrode 13 in der schematisch bei 42 angedeuteten Blickrichtung des Operateurs, so daß nach Einschalten des Hochfrequenzstromes durch Betätigen eines der hierfür vorgesehenen Betätigungsknöfe 36 die Operation bei ungehinderter Beobachtung durch den Operateur erfolgen kann. Die Beobachtung des Operationsvorganges erfolgt dabei durch ein Endoskop, welches sich entlang der Blickrichtung 42 erstrecken kann.

Statt der Feder 19 konnte auch auf der dem Zugdraht 17 diametral gegenüberliegenden Seite ein weiterer Zugdraht 18 vorgesehen werden, der bei 46 diametral der Befestigungsstelle 39 gegenüberliegend am hinteren Ende der Arbeitsspitze 12 befestigt ist. Durch gegenläufige Zugbeaufschlagung der Zugdrähte 17, 18 kann die Arbeisspitze 12 ebenfalls zwischen Einführungs- und Entnahmeposition einerseits und Arbeitsposition andererseits hin- und hergeschwenkt werden.

In den folgenden Figuren bezeichnen gleiche Bezugszahlen entsprechende Bauteile wie in Fig. 1. Das Hochfrequenzkabel 37 ist teilweise aus Gründen einer übersichtlichen zeichnerischen Darstellung nicht gezeigt, jedoch stets vorhanden.

Bei der Ausführungsform nach Fig. 2 sind zwischen dem starren Rohrschaft 11 und der Arbeitsspitze 12 mehrere hintereinandergeschaltete Gelenkglieder 16 vorgesehen, wodurch ein besonders großer Schwenkwinkel der Arbeitsspitze 12 von annähernd 90 um eine fiktive Drehachse 15' erzielt werden kann.

Zwischen dem distalen Ende des Rohrschaftes 11 und der Arbeitsspitze 12 erstreckt sich eine reibend darübergeschobene Schraubenfeder 19', welche die Arbeitsspitze 12 und die Gelenkglieder 16 in eine mit dem Rohrschaft 11 axial ausgerichtete Position vorspannt. Durch den bei 39 in einem Abstand von der Mittelachse befestigten Zugdraht 17 kann die Arbeitsspitze 12 in die aus Fig.2 ersichtliche Arbeitsposition verschwenkt werden.

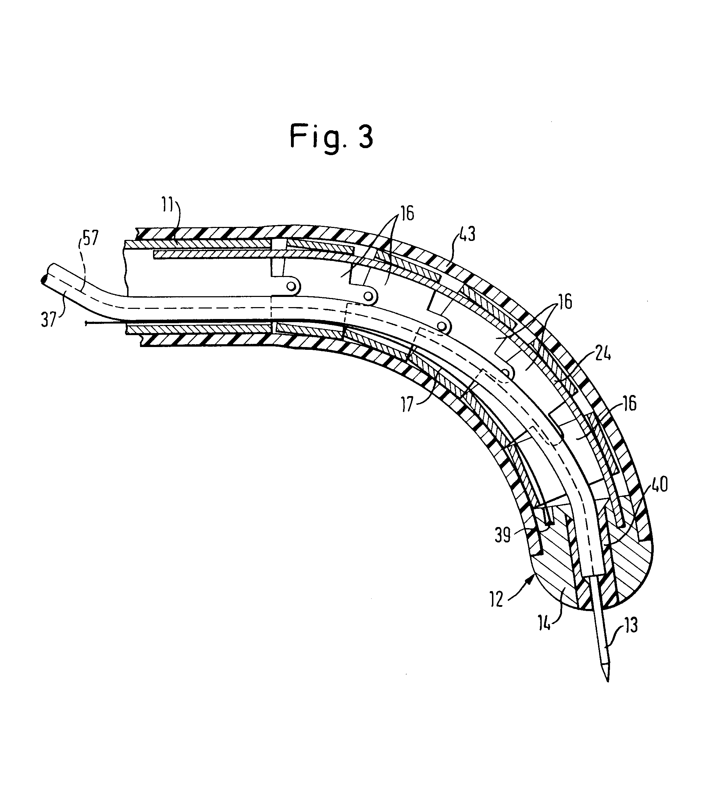

Beim Ausführungsbeispiel nach Fig. 3 ist statt der Schraubenfeder 19' nach Fig. 2 ein flexibler Schlauch 43 über die Arbeitsspitze 12, die Gelenkglieder 16 und das distale Ende des Rohrschaftes 11 geschoben, wodurch die Gelenkglieder 16 vollständig nach außen hermetisch abgeschlossen sind. Die für die axiale Ausrichtung der Arbeitsspitze 12 mit dem Rohrschaft 11 erforderliche Rückstellfederkraft könnte grundsätzlich durch einen geeignet elastisch ausgebildeten Isolationsschlauch erzeugt werden. Beim Ausführungsbeispiel nach Fig.3 erstreckt sich jedoch zu diesem Zweck zwischen dem distalen Ende des Rohrschaftes 11 und der rohrförmigen Neutralelektrode 40 der Arbeitsspitze 12 eine Blattfeder 24. Diese besorgt zusammen mit dem Zugdraht 17 das Ausstellen in die in Fig. 3 dargestellte Arbeitsposition bzw. das Rückstellen in die axial ausgerichtete Position des Rohrschaftes 11 und der Arbeitsspitze 12.

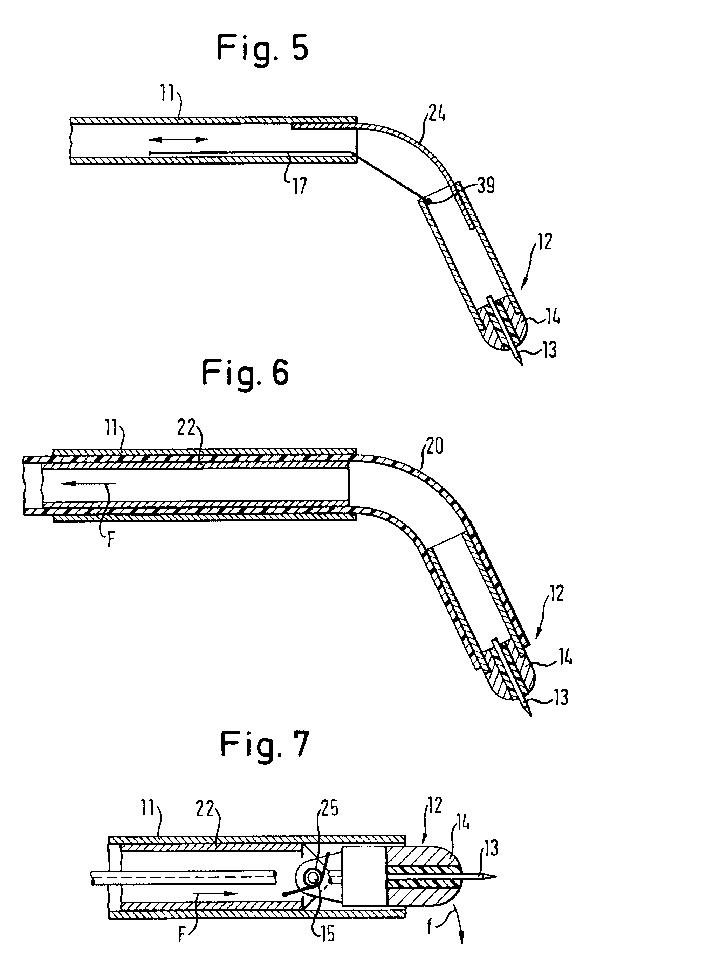

Fig. 4 zeigt eine besonders einfache Ausführungsform, bei der die Arbeitsspitze 12 auf eine entsprechend steif ausgebildete Schneidnadel 13 reduziert ist, die am distalen Ende des Rohrschaftes 11 mittels eines isolierten Lagerbockes 44 gelenkig, d.h. um eine Drehachse 15 verschwenkbar befestigt ist. Mittels eines Winkelhebels 45 und einer axial im Innern des Rohrschaftes 11 verlaufende und hin- und herverschiebbare Druck-Zugstange 21, die gleichzeitig die Hochfrequenzzufuhr besorgen, kann die Schneidnadel 13 sowohl in die aus Fig.4 ersichtliche abgewinkelte Arbeitsposition als auch in eine mit dem Rohrschaft 11 axial ausgerichtete Lage verschwenkt werden.

Aus Fig. 5 ist ersichtlich, daß eine sich zwischen Rohrschaft 11 und Arbeitsspitze 12 erstrekkende Biege-Blattfeder 24 nicht nur die Rückstellkrafterzeugung zwischen diesen beiden Elementen übernehmen kann, sondern gleichzeitig für die gelenkige Verbindung des Rohrschaftes 11 und der Arbeitsspitze 12 sorgt. Ein besonderes mechanisches Gelenk kann auf diese Weise vermieden werden.

Nach Fig. 6 ist innerhalb des Rohrschaftes 11 ein als Steuerglied wirkendes Innenrohr 22 axial verschiebbar angeordnet. Das Innenrohr 22 ist mit einem flexiblen und elastischen Isolierschlauch 20 überzogen, der über das distale Ende des Rohres 22 und des Rohrschaftes 11 vorsteht und sich dort bis über die Arbeitsspitze 12 erstreckt und diese hält. In seinem entspannten Zustand nimmt der Isolierschlauch 20 die aus Fig.6 ersichtliche um etwa 60 _{°} relativ zum Rohrschaft 11 abgebogene Arbeitsposition ein. Durch Zurückziehen des Rohres 22 in Richtung des Pfeiles F innerhalb des Rohrschaftes 11 wird der zunächst gekrümmte Teil des Isolierschlauches 20 in den geradlinigen Rohrschaft 11 hineingezogen und dadurch federnd gerade gerichtet. Sobald das hintere Ende der Arbeitsspitze 12 sich im distalen Bereich des Rohrschaftes 11 befindet, sind der Rohrschaft 11 und die Arbeitsspitze 12 axial ausgerichtet und nehmen somit ihre Einführ- und Entnahmeposition ein.

Nach Fig. 7 ist das zum Rohrschaft 11 konzentrische und als Steuerglied wirkende Innenrohr 22 ohne darübergezogenen Isolierschlauch axial verschiebbar im Rohrschaft 11 angeordnet. An seinem vorderen Ende ist über ein Gelenk 15 die Arbeitsspitze 12 verschwenkbar befestigt. Eine Schenkelfeder 25 spannt die Arbeitsspitze 12 in Richtung des Pfeiles f vor.

Befindet sich das Rohr 22 in der in Fig. 7 dargestellten am meisten zurückgezogenen Position innerhalb des Rohrschaftes 11, so ist die entsprechend dimensionierte Arbeisspitze 12 mit ihrem hinteren Ende im vorderen Teil des Rohrschaftes 11 angeordnet und mit ihm axial ausgerichtet.

Wird jetzt das Innenrohr 22 in Richtung des Pfeiles F nach vorn verschoben, so tritt die Arbeitsspitze 12 mehr und mehr aus dem vorderen Ende des Rohrschaftes 11 aus, bis es vollständig aus diesem herausragt. Nunmehr verschwenkt die entsprechend vorgespannte Schenkelfeder 23 die Arbeitsspitze 12 in Richtung des Pfeiles f nach unten bis zu einem Anschlag, so daß sie schließlich ihre Arbeitsposition einnimmt.

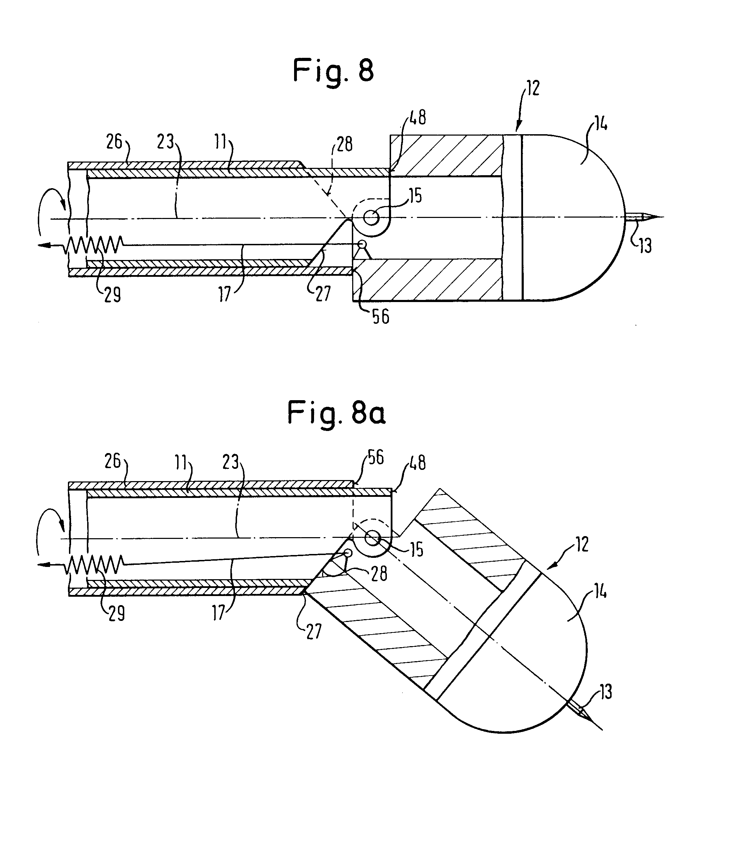

Nach Fig. 8 ist ein starres Rohr 26 außen auf dem Rohrschaft 11 drehbar, aber nicht axial verschiebbar angeordnet. Die teilweise als Anschlagfläche 46 ausgebildete vordere Stirnfläche des starren Rohres 26 ist bis zur Mittellängsachse 23 unter einem Winkel von etwa 45 _{°} abgeschrägt und bildet dort eine Schrägfläche 28.

Eine entsprechende Abschrägung 28 weist auch die teilweise als Anschlagfläche 48 ausgebildete vordere Stirnfläche des starren Rohrschaftes 11 auf, jedoch bei der Drehstellung nach Fig.8 auf der der Abschrägung der Stirnfläche 28 diametral gegenüberliegenden Seite der Mittellängsachse 23.

Sind die beiden Abschrägungen 27, 28 gemäß Fig.8 um 180 _{°} relativ zur Mittelachse 23 gegeneinander versetzt, bewirkt ein von einer Zugfeder 29 beaufschlagter Zugdraht 17 die Anlage der mit dem Rohrschaft 11 axial ausgerichteten Arbeitsspitze 12 an einer Anschlagfläche 46 am vorderen Ende des starren Rohres 26.

Wird jetzt das starre Rohr 26 relativ zur Mittellangsachse 23 um 180 in die Position nach Fig. 8a gedreht, so gelangt statt der in Fig. 8 vorgesehenen Anschlagfläche 46 die Schrägfläche 28 mit der Abschrägung 27 des Rohrschaftes 11 in Ausrichtung, wodurch die Arbeitsspitze 12 unter Einwirkung der Zugfeder 29 über den Zugdraht 17 in die aus Fig. 8a ersichtliche untere Arbeitsposition ausschwenken kann, wo sie an den ausgerichteten Schrägflächen 27, 28 anliegt.

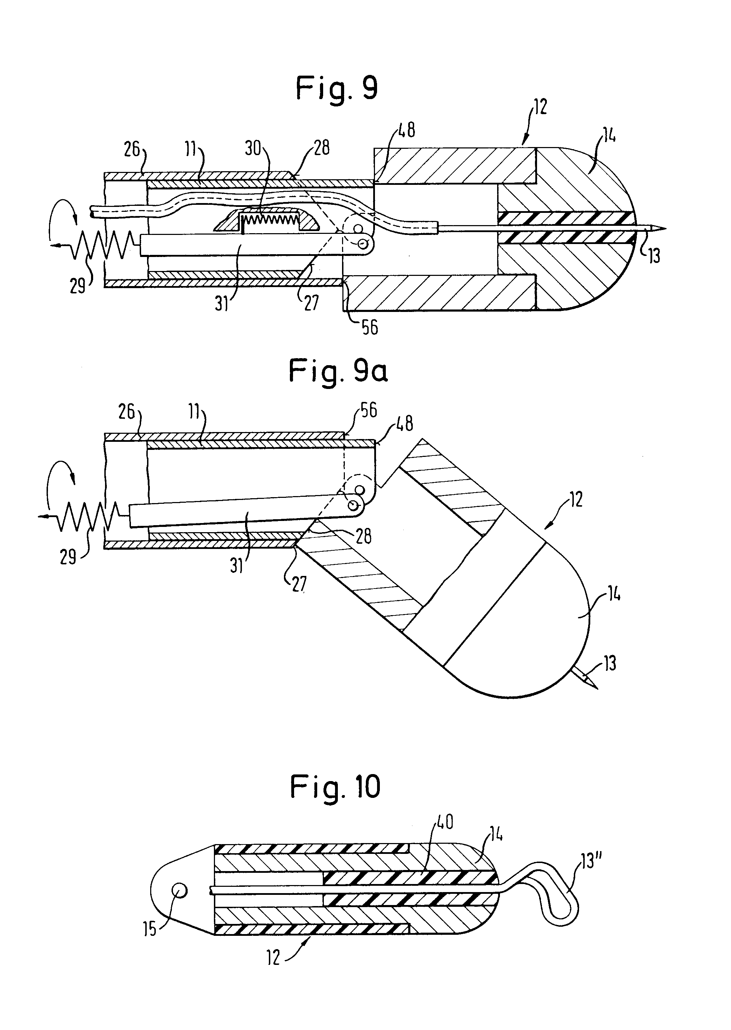

Eine ähnliche Ausführungsform zeigen die Fig. 9 und 9a, wobei lediglich statt des Zugdrahtes 17 nach den Fig. 8, 8a eine Druckstange 31 vorgesehen ist. Diese kann statt durch die Zugfeder 29 auch durch eine nur in Fig. 9 schematisch angedeutete Druckfeder 30 beaufschlagt werden.

Fig. 10 zeigt eine Arbeitsspitze 12, die an ihrem distalen Ende mit einer Schneidschlinge 13" versehen ist. Diese erstreckt sich zwar teilweise quer zur Mittellängsachse der Arbeitsspitze 12, steht jedoch radial nicht über deren Außenumfang vor, so daß sie das Einführen des Instrumentes in ein Trokar und zu einem Operationsfeld nicht behindert. Gleichwohl wird die Schneidschlinge 13" nach dem Verschwenken um die Drehachse 15 in die Arbeitsposition vom Operateur beobachtbar.

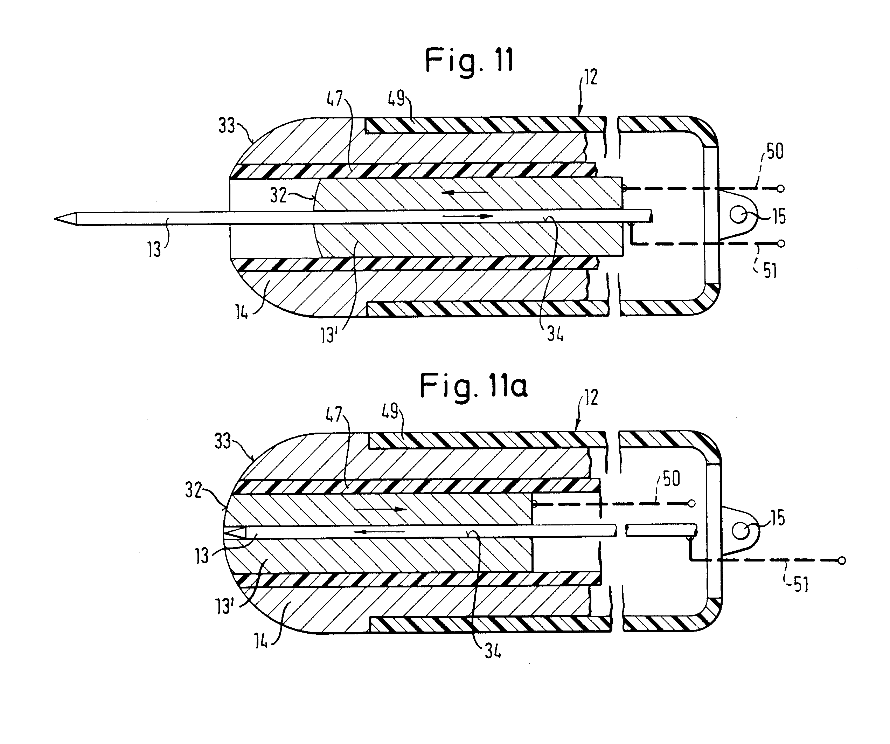

Nach den Fig. 11 und 11 weist die Arbeitsspitze 12 sowohl eine Koagulationselektrode 13' mit einer vorderen konvexen Stirnfläche 32 als auch eine Schneidnadel 13 auf. Die Neutralelektrode 14 ist wie bei allen anderen Ausführungsformen rohrförmig ausgebildet und am distalen Ende mit einer konvexen Stirnfläche 33 versehen. Zwischen der Neutralelektrode 14 und der dazu koaxialen Koagulationselektrode 13' befindet sich eine koaxiale Isolierschicht 47. Zweckmäßigerweise ist die Neutralelektrode 14 auch im Bereich hinter der Stirnfläche 33 mit einer Isolierung 49 versehen.

Erfindungsgemäß ist nun die in der Mitte vorgesehene Koagulationselektrode 32 durch einen nicht im einzelnen dargestellten und in Fig. 11, 11 a nur gestrichelt angedeuteten Betätigungsmechanismus 50 in Richtung des Pfeiles in Fig.11 nach vorn in eine in Fig. 11 a dargestellte Koagulationsposition verschiebbar, in der die Stirnflächen 32, 33 mit der dazwischen befindlichen Stirnfläche der Isolierschicht 47 eine durchgehende etwa halbkugelförmige Koagulationsfläche bilden. In dieser Arbeitsposition der Koagulationselektrode 32 ist die koaxial in einer Bohrung 34 der Koagulationselektrode 13' vorgesehene Schneidnadel 13 durch einen nur gestrichelt angedeuteten Betätigungsmechanismus 51 in Richtung des Pfeiles in Fig. 11 so zurückgezogen, daß sie nicht nach vorn über die Stirnfläche 32 der Koagulationselektrode 13' vorsteht.

Mittels des Betätigungsmechanismus 51 kann die Schneidnadel 13 jedoch in Richtung des Pfeiles in Fig. 11 nach vorn in die aus Fig. 11 ersichtliche Position vorgeschoben werden, während gleichzeitig die Koagulationselektrode 13' in die aus Fig.11 ersichtliche Lage zurückgezogen wird.

Durch Anlenken der aus den Fig. 11 und 11 a ersichtlichen Arbeitsspitze 12 über die Drehachse 15 an einen in den vorangehenden Ausführungsbeispielen dargetellten Rohrschaft 11 kann somit ein besonders hohes Maß an Universalität erreicht werden. Die Arbeitsspitze 12 kann nicht nur durch das vorstehend beschriebene Verschwenken aus der axial ausgerichteten Einführungs- und Entnahmeposition in eine Arbeitsposition verschwenkt werden, sondern es können auch noch wahlweise die Koagulationselektrode 13' oder die Schneidnadel 13 in der aus den Fig. 11 und 11 ersichtlichen Weise wirksam bzw. unwirksam gemacht werden.

### Bezugszeichenliste

11 Rohrschaft
12 Arbeitsspitze
13 Schneidelektrode
13' Koagulationselektrode
13" Schneidschlinge
14 Neutralelektrode
15 Drehachse
15' Drehachse
16 Gelenkglied
17 Zugdraht
18 Zugdraht
19 Feder
19' Feder
20 Isolierschlauch
21 Schub-Zugstange
22 Steuerglied
23 Mittellängsachse
24 Biegefederelement
25 Schenkelfeder
26 starres Rohr
27 Stirnseite
28 Stirnfläche
29 Zugfeder
30 Druckfeder
31 Druckstange
32 Stirnfläche
33 Stirnfläche
34 Bohrung
35 Handgriff
36 Betätigungselemente
37 Hochfrequenzkabel
38 Anschlagfläche
39 Punkt
40 Isolierkern
41 Drehknopf
42 Blickrichtung
43 flexibler Schlauch
44 Lagerbock
45 Winkelhebel
46 Anschlagfläche
47 Isolierschicht
48 Anschlagfläche
49 Isolierung
50 Betätigungsmechanismus

## Patentansprüche

1. Bipolares Hochfrequenz-Chirurgieinstrument mit einem starren Rohrschaft (11) und einer vorzugsweise eine im wesentlichen den gleichen Querschnitt wie der anschließende Teil des Rohrschaftes (11) aufweisenden Arbeitsspitze (12), an der vorzugsweise zentral wenigstens eine von einer Hochfrequenzspannung beaufschlagbare Arbeitselektrode, wie eine Schneidelektrode (13, 13") oder Koagulationselektrode (13') und eine vorzugsweise eine deutlich größere Oberfläche aufweisende Neutralelektrode (14) vorgesehen sind,
dadurch gekennzeichnet,
daß die Arbeitsspitze (12) nahe dem distalen Ende des Instrumentes gelenkig oder biegsam mit dem starren Rohrschaft (11) derart verbunden ist, daß sie aus einer mit dem Rohrschaft (11) axial ausgerichteten Position in eine relativ zum Rohrschaft (11) abgewinkelte Position verschwenkbar ist, und daß Mittel (17, 18, 19, 19', 20, 21, 22, 24, 25, 26, 29, 30, 31) vorgesehen sind, um die Arbeitsspitze (12) zu verschwenken und wieder zurückzustellen.

2. Instrument nach Anspruch 1, dadurch gekennzeichnet, daß die Drehachse (15, 15') zwischen dem Rohrschaft (11) und der Arbeitsspitze (12) weniger als 10 cm und vorzugsweise weniger als 5 cm sowie insbesondere 2 bis 3 cm vom vorderen Ende der Arbeitselektrode (13, 13', 13") entfernt ist.

3. Instrument nach Anspruch 1 oder 2, dadurch gekennzeichnet, daß die Arbeitsspitze (12) durch ein Drehgelenk (15) am starren Rohrschaft (11) befestigt ist.

4. Instrument nach Anspruch 1, dadurch gekennzeichnet, daß durch mehrere rohrförmige Gelenkglieder (16) ein quasi biegsamer Abschnitt zwischen dem starren Rohrschaft (11) und der Arbeitsspitze (12) gebildet ist.

5. Instrument nach Anspruch 1, dadurch gekennzeichnet, daß zwischen dem Rohrschaft (11) und der Arbeitsspitze (12) wenigstens ein Biegefederelement (24) angeordnet ist.

6. Instrument nach Anspruch 5, dadurch gekennzeichnet, daß das oder die Biegefederelemente (24) exzentrisch zur Achse des Rohrschaftes (11) angeordnet sind.

7. Instrument nach Anspruch 5 oder 6, dadurch gekennzeichnet, daß das Widerstandsmoment des bzw. der Biegefederelemente (24) in der Biegeebene mindestens dreimal größer ist als in der senkrecht dazu stehenden Ebene.

8. Instrument nach einem der Ansprüche 5 bis 7, dadurch gekennzeichnet, daß das Biegefederelement bzw. die Biegefederelemente als Blattfeder bzw. Blattfedern (24) ausgebildet ist bzw. sind, die mit ihrer flachen Seite zur Mittellängsachse des Rohrschaftes (11) weisen.

9. Instrument nach einem der vorhergehenden Ansprüche, dadurch gekennzeichnet, daß die Mittel zum Verschwenken und Zurückstellen der Arbeitsspitze umfassen:
- zwei exzentrisch angeordnete, gegenüberliegende Zugdrähte (17, 18) oder
- eine exzentrisch zur Drehachse (15, 15') angeordnete Rück- oder Ausstellfeder (19, 19', 25, 25, 29, 30) und ein diametral gegenüberliegendes Zugglied (17) oder
- eine symmetrisch angeordnete Schraubenfeder (19') und ein Zugglied (17) oder
- ein Verbindungselement (20) zwischen Rohrschaft (11) und Arbeitsspitze (12) aus elastischem Werkstoff und ein Zugglied (17) oder
- eine Schub- und Zugstange (21) oder
- ein im Rohrschaft (11) axial verschiebbares Steuerglied (22), welches die Arbeitsspitze (12) schwenkbar (23) und relativ zum Rohrschaft (11) ausstellbar trägt.

10. Instrument nach einem der Ansprüche 1 bis 8, dadurch gekennzeichnet, daß zwischen Arbeitsspitze (12) und starrem Rohrschaft (11) ein Drehgelenk (15) angeordnet ist, der starre Rohrschaft (11) an seiner distalen Stirnseite mindestens bis zur Mittelachse abgeschrägt (27) ist, daß ein zweites Rohr (26) mit abgeschrägter (28) Stirnfläche verdrehbar und konzentrisch zum starren Rohrschaft (11) angeordnet ist und ein vorzugsweise durch eine Zugfeder (29) vorgespannter Zugdraht (17) oder eine durch eine Zug- oder Druckfeder (30) vorgespannte Druckstange (31) so an die Arbeitsspitze (12) angelegt ist, daß an dieser ein Drehmoment in Richtung auf die Schrägfläche (27) des Rohrschaftes (11) anliegt.

11. Instrument nach einem der vorhergehenden Ansprüche, dadurch gekennzeichnet, daß das Federelement (19, 19', 24, 25, 29, 30) in Ausstellrichtung vorgespannt ist und die Arbeitsspitze (12) an ein Steuerglied (22) angelenkt (15) ist, welches innerhalb des Rohrschaftes (11) axial verschiebbar ist.

12. Instrument nach einem der vorhergehenden Ansprüche, dadurch gekennzeichnet, daß das Federelement aus einem Federstahl, einer Federbronze oder einer superelastischen Ni-Ti-Legierung besteht.

13. Instrument nach einem der vorhergehenden Ansprüche, dadurch gekennzeichnet, daß die Arbeitselektrode als bipolare Schneidschlinge (13") zum Abtragen von Biogewebe ausgebildet ist.

14. Instrument nach Anspruch 13, dadurch gekennzeichnet, daß die Schneidschlinge (13") seitlich abgebogen ist und der von ihr isolierte metallische Schaft als großflächige Neutralelektrode (14) geformt ist, deren Fläche vorzugsweise mindestens fünfmal größer ist als die Oberfläche der Schneidschlinge (13").

15. Instrument nach einem der Ansprüche 1 bis 12, dadurch gekennzeichnet, daß die Arbeitsspitze (12) als bipolare Schneidnadel (13) ausgebildet ist, unmittelbar hinter der rundum eine großflächige Neutralelektrode (14) vorgesehen ist.

16. Instrument nach einem der Ansprüche 1 bis 12, dadurch gekennzeichnet, daß die Arbeitsspitze (12) als kombiniertes bipolares Schneid-und Koagulationselement ausgebildet ist.

17. Instrument nach Anspruch 16, dadurch gekennzeichnet, daß konzentrisch zueinander angeordnete, voneinander isolierte Koagulations-und Neutralelektroden (13', 14) vorgesehen sind, deren konvex gekrümmte Stirnflächen (32, 33) eine einheitliche Fläche bilden, daß die Koagulationselektrode (13') innerhalb der rohrförmig ausgebildeten Neutralelektrode (14) versenkbar ist und daß durch eine axiale Bohrung (34) innerhalb der Koagulationselektrode (32) eine Schneidnadel (13) herausschiebbar ist.

18. Instrument nach einem der vorhergehenden Ansprüche, dadurch gekennzeichnet, daß die Schneidnadel (13) aus einem Wolframdraht, einem Federstahldraht oder einer Ni-Ti-Legierung besteht.

19. Instrument nach einem der vorhergehenden Ansprüche, dadurch gekennzeichnet, daß die Arbeitsspitze (12) in der Arbeitsposition einen Winkel von wenigstens 200, vorzugsweise 30 bis 45 und insbesondere von etwa 35 mit der Achse des starren Rohrschaftes einschließt.
